# EUROPEAN PATENT APPLICATION

(11) **EP 3 675 137 A1**
(43) Date of publication of application: **01.07.2020**
(21) Application number: 18382995.1
(22) Date of filing: 27.12.2018
(51) Int. Cl.: G16H 50/30, G16H 10/20

(54) **METHOD AND SYSTEM FOR MORE EFFICIENT WELLBEING ESTIMATION AND IMPROVEMENT**

(71) Applicant: Telefonica Innovacion Alpha S.L, 28050 Madrid (ES)
(72) Inventor: HARRISON, Oliver, 28050 MADRID (ES); MATIC, Aleksandar, 28050 MADRID (ES)
(74) Representative: Herrero & Asociados, S.L.

(57) **Abstract**

A method and system for efficient estimation of the subjective wellbeing (SWB) score of an user and for efficient generation of user's recommendations to improve the SWB score. In order to do so, the proposed method and system uses a range of continuously captured signals (user inputs, body signals, signals captured by wearable devices, ambient and/or environmental sensors...) and applies a novel mechanism to quantify user's surrounding factors in order to generate a subjective wellbeing (SWB) score and derive a wellbeing management model that aims to improve SWB score.

## Description

### Field of the invention

The present invention has its application within the telecommunications sector, more specifically, relates to the deployment of tools using electronic devices and communication electronic devices (e.g., mobile user terminals such as smartphones or tablets or for wearable electronic devices, computers, etc.) which measure and send user's parameters and/or interact with the user, to estimate and improve his/her wellbeing. More particularly, the present invention relates to a method and electronic system for an efficient measurement, modelling and estimation of a user's well-being, which allows to derive accurate recommendations to improve the user's well-being.

### Background of the invention

Since 2008, the leading cause of death in the United States has become personal choices (smoking, bad diet habits, drinking alcohol...), and the same trend is seen in many countries worldwide.

In this context, behaviour change programs which address such personal choices have the potential to deliver considerable benefits to both the general health of the population and to the cost burden of disease across the population. However, for many people changing health-related behaviours is very challenging. Oftentimes individuals are instructed by their doctors to keep certain psychological and/or physiological parameters (heart rate, blood pressure, blood sugar level, respiratory rate, respiratory depth, stress, ... , etc.) within certain boundaries, but it is typically left to the individual to figure out the triggers that result in their non-healthy physiological parameters.

Another reason for the limited impact of behaviour change programmes to date is that advice from healthcare professionals and public health campaigns is not personalised for each user to maximise effectiveness.

Studies show that well-being improvement efforts are more effective when they are personalised to the unique environment, characteristics and behaviour of the user. Technological advancements have brought many methods for profiling individuals according to pre-defined criteria, and for automatically detecting user's activities, behaviours and states. These advancements have been exploited by a host of services typically for providing targeted content to users aiming to persuade them to purchase a product or service (advertising), however there are only a few that target specific users' well-being. These approaches either rely on the user segment or on a set of activities in order to suggest beneficial actions towards improving mood or subjective well-being.

Furthermore, changing health-related behaviours for long-term wellbeing benefits is very challenging as people typically perceive that the behavioural change will come with investing effort (such as doing sports) and/or sacrificing immediate pleasure (such as refraining from alcohol, tobacco, specific food, etc) in short-term. For that reason it is beneficial for behavioural change programs to consider perception of one's happiness and wellbeing -behavioural intervention will be more successful if it aims to maintain one's happiness for which monitoring wellbeing becomes crucial.

Wellbeing of a person, also known as subjective wellbeing (SWB) is a well-known term which can be defined as the degree to which people have positive thoughts and feelings about their lives. In other words, subjective wellbeing represents a self-evaluation of individual's life or experience. It includes reflective cognitive evaluations, such as life satisfaction and work satisfaction, interest and engagement, and affective (momentary) reactions to life events, such as joy and sadness.

The measurement or estimation of this SWB is often based on self-reports. Two components of SWB have been extensively covered by the wellbeing literature:
1) reflective wellbeing ("life-satisfaction"), that is a semi-static component, and
2) experiential wellbeing (how they feel in the moment), that is a dynamic component.

It can be considered that both components have two orthogonal dimensions to be measured (and inferred through a SWB model):
- Hedonic wellbeing (a user's subjective sense of pleasure)
- Eudemonic wellbeing (a user's subjective sense of purpose)

Wellbeing is a self-evaluation and a subjective measure, therefore it is challenging to infer it in a passive way, on the other hand in order to engaging develop wellbeing recommender services it is important to enable passive ways to accurately approximate dynamics of SWB to decrease the burden of frequently asking users to evaluate their life (life-satisfaction) and how they feel in the moment (experiential wellbeing). Asking users these questions frequently is also considered to be an intervention, with an unpredictable outcome for different kind of users. Moreover, decreasing the frequency of asking questions to users implies saving communication (e.g. bandwidth) and electronic resources (e.g. mobile phone battery) that are used for asking said questions (as they are usually made using electronic communication devices as for example a mobile phone).

Therefore a need exists for a method and apparatus for estimating the subjective wellbeing of an user in an accurate, personalised and resources saving way, minimizing at the same time the interaction with the user (e.g. minimizing the frequency of questioning the user) and allowing to derive accurate recommendations for behaviour modification to improve the user's wellbeing (avoiding undesired behaviours in the future).

### Summary of the invention

The problems found in prior art techniques are generally solved or circumvented, and technical advantages are generally achieved, by the disclosed embodiments which provide a method and system for efficient measurement, estimation of a user's subjective wellbeing (SWB) score, for well-being management and recommendation. The proposed method and system perform user data collection, data analysis, recommendation engine, modeling SWB score and deriving recommendations. Thanks to the proposed invention, the link between SWB and behavioural and surrounding factors will be understood and modeled, and ultimately the user's wellbeing will be understood.

The proposed solution builds a wellbeing management model (WBMM) based on the analysis of a set of dynamic and static parameters describing individual's behaviours, states and characteristics, sensed automatically or reported by the user. The WBMM is developed by using well-being indexes and activities, behaviours, states and characteristics or a sub-set of these. The WBMM is derived also from the data about activities, behaviours, states and similar characteristics derived from data from other users. The proposed solution may also deliver feedback and/or recommendations to the user (for example, through a user's interface) for improving user's well-being, for example, in the form of text, speech, image or video.

In a first aspect, it is proposed a computer implemented method for efficiently estimating and improving the wellbeing (subjective wellbeing) state of a user belonging to a group of users (persons), the method comprising:
a) A first electronic device receiving (through a communications network) data (e.g. continuous signals) related to the user acquired by one or more electronic devices, said data being at least one of: ambient data related to a location of the user, environmental data related to the location of the user, physiological data of the user's body and/or behavioural data of the user;
b) The first electronic device obtaining statistic parameters characterising said acquired data related to the user (e.g. parameters characterising the statistic distribution information of the data related to the user during a certain period of time and percentile information dynamically observed in the user's data), said statistic parameters being extracted by processing the received data related to the user (step a)) corresponding to said certain period of time;
c) The first electronic device applying at least one probabilistic model to said extracted statistic parameters to obtain an estimated wellbeing score for the user. In a preferred embodiment, two models will be applied, one model to estimate the semi-static component of the wellbeing and one model to estimate the dynamic component of the wellbeing.
d) Determining, using artificial intelligence (artificial intelligence techniques performed by an artificial intelligent module), a recommended range of values for parameters related to the user (a range of value for the data related to the user) based at least on the statistic parameters obtained in step b) and on the estimated wellbeing score of the user obtained in step c). This recommended range of values typically will result on an improvement of the wellbeing score of the user (and that's why they are recommended to the user). This determining step is usually performed also on the first electronic device but it may be performed in a different electronic device.

In a preferred embodiment, the probabilistic models (step c)) are generated and adjusted based on periodically received (through a communications network, e.g. by the first communications device) wellbeing information of all the users of the group, directly obtained from each user of the group through an user interface of a communications device of each user.

For directly obtaining wellbeing information of all the users of the group the following steps are followed: periodically presenting to each user of the group, through the user interface of the communications device of each user, a questionnaire previously received in the communications device of each user; and receiving through the user interface of the communications device of each user, answers to said questionnaire by each user of the group. In an embodiment said answers (wellbeing information) can be sent to the first electronic device by each communications device of each user (to adjust the probabilistic models). The questionnaire is presented to the user (to directly obtain wellbeing information)
with a certain period (a year, month, week...); in any case a period much longer than the period on which user's related data is received (step a)). In other words, the wellbeing information directly obtained from the users is obtained with much less frequency than the other type of user's related data.

In a preferred embodiment, also data related to all the users of the group (captured using electronic devices) will be used to generate said models. Said data being at least one of: ambient data related to a location of the users, environmental data related to the location of the users, physiological data of the users' body and/or behavioural data of the users. In an embodiment, parameters characterising the statistic distribution information of all the users' (of the group) data will be used to generate the semi-static wellbeing component model and percentile information dynamically observed in all the users' (of the group) data will be used to generate the dynamic wellbeing component model.

In an embodiment, also personal characteristics of all the users of the group (captured using electronic devices) will be used to generate said models.

The communications device of each of the users of the group maybe one of the following: a laptop, a tablet, a personal computer, a portable computer, a mobile phone, a smart phone...or any other electronic communications device.

In an embodiment, the statistic parameters extracted in step b) are of at least two types: parameters characterising the statistic distribution of the data related to the user during said certain period of time and at least one vector of percentiles values dynamically characterising the data related to the user. Usually a smaller period is used for the percentiles (e.g. a day, half a day) than for the statistic distribution (e.g. a week).

In this embodiment, a semi-static wellbeing score and a dynamic subjective well-being score is estimated; the estimated semi-static wellbeing is obtained applying a first probabilistic model (semi-static wellbeing component model) to the parameters characterising the statistic distribution of the data related to the user during said certain period of time and the estimated dynamic wellbeing is obtained applying a second probabilistic model (dynamic wellbeing component model) to one or more vector of percentiles values dynamically characterising the data related to the user.

In an embodiment, the first electronic device is a remote server and the one or more electronic devices acquiring the data related to the users, send through a communications network the acquired data to the server. In another embodiment, the first electronic device is an user's communications device and the one or more electronic devices acquiring the data related to the users (different from the user's communications device) send through a communications network the acquired data to the user's communications device.

In a preferred embodiment, data belonging to the fourth types of data: ambient data related to a location of the user, environmental data related to the location of the user, physiological data of the user's body and behavioural data of the user, are acquired and received by the first electronic device in step a) and used in the rest of steps. In other embodiments, only data belonging to one, two or three of these types of data is used.

Optionally, the recommended range of values for parameters related to the user (obtained in step d)) is sent by the first electronic device to the user's communication device through the communications network.

Personal characteristics of the user may be also taken into account in step c) by the one or more probabilistic models to obtain the estimated wellbeing score of the user and/or are also taken into account in step d) to determine the recommended range of values for parameters related to the user. In an embodiment, personal characteristics include at least one of the following: static or semi-static attributes assigned to a user referring to a user segment, personal traits, self-esteem, tolerance of uncertainty or any other type of personality characteristics. The personal characteristics can be obtained using any known mechanisms, for example, they may be inferred from the direct input from the user using questionnaires and surveys and defined scales and inventories, they may be obtained applying pre-defined models to user's data obtained from sensors...

In an embodiment, the environmental data are values of at least one of the following parameters: pollution, outdoor light level, light level, weather, humidity, outdoor temperature (or any other environmental factor affecting/related to the user); the behavioural data are values of at least one of the following parameters; mobility, social interactions, sleep state (or any other behavioral parameter of the user); the physiological data are values of at least one of the following parameters of the user's body: galvanic skin response, heart rate variability, skin temperature (or generally speaking any measurement of any physical parameter of the user's body); and the ambient data are values of at least one of the following parameters: indoor temperature, indoor light level, light exposure, noise level (or any other ambient parameter affecting/related to the user).

In an embodiment, the electronic devices acquiring data signals related to the user are at least one of the following: usage logs or sensors embedded in a communications device of the user, wearable electronic devices with sensors to measure body signals, presence sensors, location sensors, smart environments devices, bed-sensors, environment sensors to monitor environment parameters such as temperature, air quality or weather information...

In a second aspect, it is proposed an electronic system for efficiently estimating and improving the wellbeing state of a user of a group of users, the system comprising:
- Communication means (i.e. a communications module) for receiving, through a communications network, data related to the user acquired by one or more electronic devices, said data being at least one of: ambient data related to a location of the user, environmental data related to the location of the user, physiological data of the user's body and/or behavioural data of the user;
- Processing means configured to:
   - Obtain statistic parameters characterising said acquired data related to the user, the statistic parameters being extracted by processing the data related to the user corresponding to a certain period of time;
   - Apply at least one probabilistic model to said extracted statistic parameters to obtain an estimated wellbeing score for the user;
   - Based at least on the extracted statistic parameters characterising the data related to the user and the estimated wellbeing score of the user, determine using artificial intelligence a recommended range of values for parameters related to the user (which will result on an improvement of the wellbeing score of the user).

In a preferred embodiment, the communication means are further configured to receive through the communications network: wellbeing information of all the users of the group (directly obtained from each user of the group through an user interface of a communications device of each user), data related to all the users of the group and optionally personal characteristics of all the users of the group; and where the at least one probabilistic model is generated and adjusted by the processing means based on the received wellbeing information of all the users of the group, on the received data related to all the users of the group and, optionally, on the personal characteristics of all the users of the group.

A last aspect of the invention refers to a computer program product comprising computer program code adapted to perform the method of the invention, when said program code is executed on a computer, a digital signal processor, a field-programmable gate array, an application-specific integrated circuit, a microprocessor, a micro-controller, or any other form of programmable hardware. A non-transitory digital data storage medium is also provided for storing a computer program which comprises instructions causing a computer executing the program to perform the above-described method.

### Description of the drawings

For the purpose of aiding the understanding of the characteristics of the invention, according to a preferred practical embodiment thereof and in order to complement this description, the following figures are attached as an integral part thereof, having an illustrative and non-limiting character:
Figure 1 shows a schematic graphic of the extraction of distribution of longitudinally monitored indexes according to an embodiment of the present invention.
Figure 2 shows a schematic graphic of the quantification of dynamic values of longitudinally monitored indexes according to an embodiment of the present invention.
Figure 3 shows a block data flow of the development of semi-static SWB models according to an embodiment of the invention.
Figure 4 shows a block data flow of the development of dynamic SWB models according to an embodiment of the invention.
Figure 5 shows a block data flow of the development of the well-being management model according to an embodiment of the invention.

### Detailed description of embodiments of the invention

The matters defined in this detailed description are provided to assist in a comprehensive understanding of the invention. Accordingly, those of ordinary skill in the art will recognise that variation changes and modifications of the embodiments described herein can be made without departing from the scope of the invention. Also, description of well-known functions and elements are omitted for clarity and conciseness.

Note that in this text, the term "comprises" and its derivations (such as "comprising", etc.) should not be understood in an excluding sense, that is, these terms should not be interpreted as excluding the possibility that what is described and defined may include further elements, steps, etc.

Of course, the embodiments of the invention can be implemented in a variety of architectural platforms, operating and server systems, devices, systems, or applications. Any particular architectural layout or implementation presented herein is provided for purposes of illustration and comprehension only and is not intended to limit aspects of the invention.

The proposed embodiments provide a method and system for efficient measurement and estimation of the subjective wellbeing (SWB) score of a user (a person) and for efficient generation of user's recommendations to improve the SWB score. In order to do so, the proposed method and system uses a range of continuously captured signals (user inputs, body signals, signals captured by wearable devices, ambient and/or environmental sensors...) and applies a novel mechanism to quantify user's phenotype and surrounding factors in order to generate a subjective wellbeing (SWB) score and derive a wellbeing management model that aims to improve SWB score.

More specifically, the described embodiments proposed a new way of inferring SWB in a passive way by creating a model that maps behavioural, body, ambient and environmental signals to SWB through analysis of distributions of these signals to model semi-static component of SWB (e.g. reflective wellbeing) and through calculating percentiles (for a shorter time frame) of these distribution for modeling dynamic component of SWB (e.g. experiential wellbeing).

In the prior art solutions, the user was frequently asked to evaluate his/her wellbeing (e.g. life-satisfaction and experiential wellbeing). However, as previously stated in the "Background" section, these frequently asking imply several drawbacks (included a higher resources expenditure). In order to avoid these drawbacks, in the proposed method and system the SWB score is inferred in a passive way by creating a model based on behavioural, body, ambient and environmental information. In the proposed embodiments, the frequency of asking the user's is significantly decreased compared to the prior arts as the user's is only directly asked (using an electronic communications device) in order to get ground-truth information and to occasionally calibrate the system (the term "ground-truth" refers to the process of gathering the proper objective data for the learning process or in other words, the "ground-truth" will be the information provided by direct observation, i.e. empirical evidence, as opposed to information provided by inference).

Hence, in the proposed system and method SWB is inferred in a passive way and to derive wellbeing management model through inferring the main drivers of SWB. This not only avoid the prior art drawbacks previously mentioned but it is important also both for individuals and for medical practitioners to understand better consequences (positive and negative) of specific behaviours and intertwined effects of behaviours, and surrounding factors.

The proposed system monitors (measures) several parameters (also called factors) which may depend on or may affect the user's subjective wellbeing. For example, environmental factors (such as pollution, outdoor light level, light level, weather, humidity, outdoor temperature or any other environmental factor), sensed behaviours (such as mobility, social interactions, sleep state ...), body signals (such as galvanic skin response, heart rate variability, skin temperature or generally speaking any measurement of any physical parameter of the user's body), and/or ambient parameters (indoor temperature, indoor light level, light exposure, noise level and any other ambient parameter). This is a non-limitative list and other type of parameters can be monitored and used.

In a preferred embodiment, all the four types of parameters are used (environmental, sensed behaviours, body signals or ambient parameters). In other embodiments, only one or several of these type of parameters are used (generally speaking the more type and amount of parameters used, the more accurate the estimation of the SWB score will be but also the more resources will be spent).

The system may also use captured information directly from users (through questionnaires, and surveys, about their traits, demographics, and SWB measurements). However, as stated before, the type of parameters obtained (only to get ground-truth information or to calibrate the system) directly by asking the user are obtained with much less frequency than the other type of parameters.

The parameters monitored (e.g. continuous signals) and used by the proposed system are captured from one or more sources of data (that is, using one or more electronic devices which obtain the value of said parameters), as for example (this is a non-limitative and other sources of data may be used):
- In order to ask questions to the user (e.g. in the form of a survey or a questionnaire) and to receive direct user inputs (e.g. answers to the questions asked to the user) a communications device (also called user's communications device or simpler, user's device) is used. The users' communications device may be any electronic communication device as for example a laptop, a tablet, a personal computer, a portable computer, a mobile phone, a smart phone, any personal communications device or, generally speaking, any electronic communications device. The user's communications device must have an electronic user's interface (for example, a screen, a keyboard, a speaker, a microphone...) in order to be able to asks the questions to the user and receive his/her answers.
- Usage logs or sensors embedded in personal devices (for example hand-held devices) such as wearable devices, hand-held devices as mobile phones, smart phones, tablets, laptops, ... and similar. This device may be the user's communications device employed to ask questions and receive inputs of the user or a different device. That is, the user's communication device (for example, his/her mobile phone) can be used also to monitor other parameters besides direct user inputs (for example, body signals of the user as heart rate, ambient parameters as light level, environmental factors, behaviour parameters as social interactions...).
- Dedicated body sensors to measure body signals. Said dedicated body sensors may be included in wearable electronic devices as for example smart-watches, bracelets (e.g. fitbit, jawbone...), clips (e.g. fitbit) or any other type of wearable electronic devices which can be worn by the user.
- Ambient (indoor) sensors such as presence sensors, location sensors, smart environments devices, bed-sensors, etc.
- Environment (outdoor) sensors to monitor environment parameters such as temperature, air quality, weather information and any other environment parameters.
- External sources (such as dedicated data-base, news, social networks, ...)
- And generally speaking any known electronic device which may be used to obtain the value of the parameters used by the system. As it can be seen, a single electronic device can be used to obtain the value of different type of parameters (for example, a wearable device can be used to obtain body signals, but also environmental factors...or the mobile phone can be used to obtain direct inputs from the user but also body signals, ambient parameters, environmental factors, behaviour parameters...

All of this data captured over time for a specific user (that's why is also called longitudinal data) that refers to one specific user is stored in a database (called user database). That is all the parameter values obtained by the different electronic devices are sent by said electronic devices to a database. This data base may be internal to one of these electronic devices (for example, the user's communications device) or may be external to all the electronic devices used to obtain this data (for example, in a remote server). The electronic devices used to obtain this longitudinal data should have a communication interface which allows the electronic device to communicate the obtained data to the database (directly or through another electronic device) over a communications network. The communications network may be a wireless communications network, a mobile communications network as GSM 2G, GPRS, UMTS, 3G, 4G or LTE, LAN or W-LAN or any other type of wired or wireless communication networks.

For simplicity, in the above embodiment, it is supposed that all the captured data is stored in a single database but generally speaking the data can be stored in one or more databases in the same or different electronic devices.

In a preferred embodiment, the data of the user database (captured by the different data sources as user inputs in the user's communications device, hand-held devices, wearable devices, ambient sensors, environmental monitors...) are not used as such but are processed using computer based techniques to obtain more usable, quantifiable and representative data (called indexes or indices). In order to do that the data of the user database are fed into different modules to obtain a set of indexes. These modules are functional blocks (performed by a computer processor) which apply a certain model to obtain said indexes. The applied model will depend on the type of parameters processed (externally developed models, pre-defined models, can be used). For example, a behaviour recognition model will be applied to the behavioural parameters from the data base to obtain behavioural indexes, a physiological state model will be applied to the body parameters from the data base to obtain body (physiological) index, an ambient sate model will be applied to the ambient parameters from the data base to obtain ambient indexes and/or an environmental state model will be applied to the environmental parameters from the data base to obtain environmental indexes. This is a non-limitative list and other type of parameters and, consequently, of models can be used.

In a preferred embodiment, all the four type of parameters are used so all the models will be used. In other embodiments, only one or several of these types of parameters are used so only some of these models will be applied.

Any model can be used to obtain usable, quantifiable and/or representative user information indexes from the data on the user database (known models will be usually used). For instance, behaviours may be defined as patterns of the activities of daily living (such as eating, sleeping, doing sports, socializing, etc.). The system may infer activities or represents behaviours using the direct input from the user and/or based on the output from any of the electronic devices previously mentioned (for example, sensors). The system uses said pre-defined models of converting one or more signals (raw data) to behaviour patterns (and from the patterns, the behavioural indexes are obtained). In the case of physiological data, physiological states may represent a dynamically changing information about a user (such as heart rate variability or galvanic skin response) which is often linked to emotional state, mood. The system infers states from the direct input from the user and/or based on the output from any of the electronic devices previously mentioned (for example, sensors). The system uses the pre-defined models of converting one or more signals to physiological states.

Personal characteristics (also called personality characteristics) may include static (or semi-static, i.e., slowly changing) attributes assigned to a user referring to a user segment (e.g., demographics), personal traits (e.g., personality, boredom proneness), self-esteem, tolerance of uncertainty or any other type of personality characteristics. The personal characteristics can be for example inferred from the direct input from the user using questionnaires and surveys and defined scales and inventories or can be obtained applying models to data acquired by sensors. The system uses the pre-defined models of converting one or more signals into personal characteristics and produces scores that then represent the attributes associated with the specific user. Typically, these personal characteristics are more static (i.e. less variable) that the environmental, behavioural, body or ambient parameters.

The user data (as stored in the database or the obtained indexes if the previously explained process of the data stored in the databases is performed) are further processed (by a computer processor) in order to obtain specific input for the SWB models. More specifically, this further process is made to obtain a semi-static representation (or component) of the user data and a dynamic representation (or component) of the user data. As it will be explained now, the semi-static representation of the user data will be based on characterisation of distribution of the data and a dynamic representation of the user data is a current value represented as a percentile of the said distribution of the data.

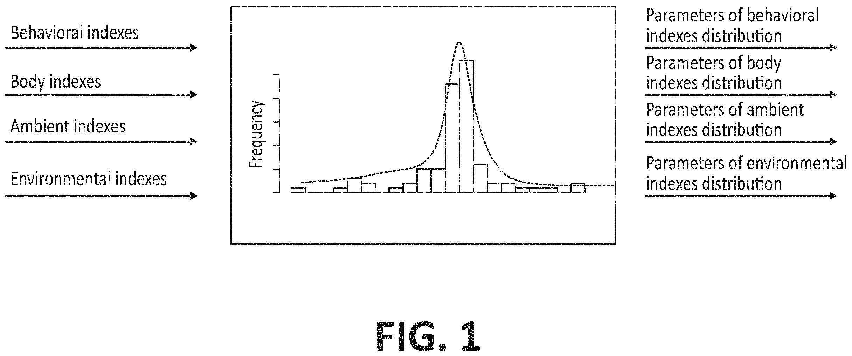

In a preferred embodiment, as part of this process, the user behavioural, body, ambient and/or environmental data (e.g. indexes) are processed to extract its (statistic) distribution (as shown in figure 1). That is, in order to model semi-static parameters of user's phenotype and the surrounding factors, each of the indexes (each type of data) is analyzed through its distribution of values (e.g. distribution of frequency of each value as shown in figure 1) collected longitudinally (that is, over a certain period of time eg. a week, a month, a year...). This step includes extraction of distribution parameters, and in this way each index (behavioural, body, ambient and/r, environmental) will be converted into a set of statistic distribution parameters. For instance, if it is a near-normal distribution these distribution parameters will include mean and standard deviation, for power law distribution this would include Xmin and a, etc, followed by the extraction of Kolmogorov-Smirnov coefficients about the distribution fit. The goal is to quantify typical patterns of a specific index per user, and quantify baseline behaviours and surrounding factors. These semi-static parameters will be used to model the semi-static SWB component.

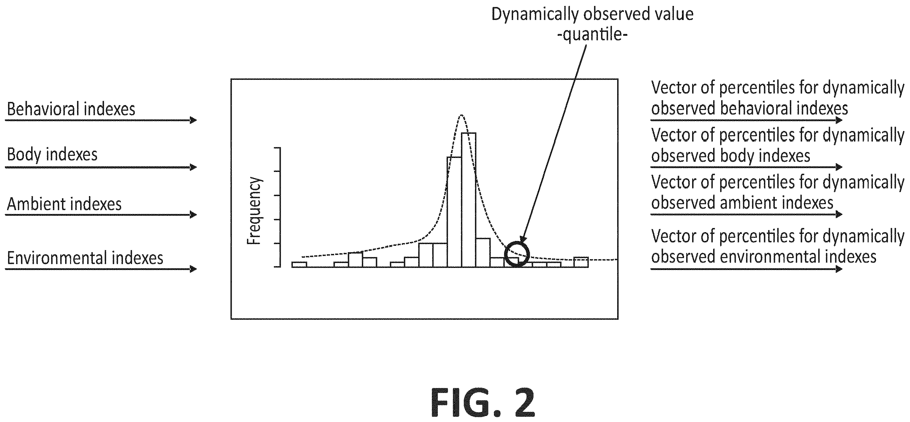

Other part of this process is to quantify dynamic behavioural and surrounding parameters. In order to do so, considering the range of longitudinal values (i.e., the previously described distribution), dynamic values observed in a specific time period (such as, part of a day, day, weekend, week, month or any other) are delivered to the model to obtain an estimation of the dynamic SWB (as it will be later explained) not as absolute values but as percentiles of the respective distributions (e.g. distribution of frequency of each value as shown in figure 2). For example, consider a situation in which a user makes 8000 steps a day, with an average speed of 6km/h, and that over time both speed and number of steps at a daily level follow a normal distribution during a period of the user's life in which the system has been monitoring him/her (i.e. assuming that the data from a certain number of days have been collected). If today the user made 1000 steps with a speed of 8km/h, these dynamic values can be expressed as a percentile of the user's typical distribution and say e.g. that the user's today number of steps belongs to 25^{th} percentile (lower than 75% of the user's days) but the user's speed belongs to 90^{th} percentile (in top 10% of the user's days).

Using percentiles instead of absolute values when it comes to extracting features (i.e. parameters) that are used as an input into the models, is a very novel approach. This is the advantage that resolves the drawbacks of similar models that suffer from the typical approach of using absolute values (e.g., daily mean of heart-rate, sleep hours, daily time at home/at work, daily time with friends, daily ambient temperature, etc.) as input parameters, as they don't capture individual differences well (e.g., an outlier for a user 1 may mean a mean value for a user 2). Therefore, using percentiles instead of absolute values, the method captures more accurately a differentiation between users with less captured data (so resources are saved). So, in this part of the process, the user behavioural, body, ambient and/or environmental data (e.g. indexes) are processed to extract the percentiles of the distributions of each captured parameter (as shown in figure 2). In a preferred embodiment, a vector of percentiles is extracted for each type of parameters (behavioural, body, environmental, ambient...). The dimension of the vector will depend on how many parameters (variables) are monitored for each of the types; for example, if the environmental parameters monitored (captured) are pollution level, temperature and humidity, the vector of percentiles of the environmental parameters will have a dimension=3. The exact percentiles values will depend on the distribution of the parameter values observed in a certain period of time. For example, considering again the above example for steps and walking speed, if the user's wearable (e.g. fitbit) observes that today he/she has made 1000 steps and that it belongs to 25^{th} percentile with respect to distribution of daily steps in his/her life, this 25^{th} percentile will be an observed value for today. Also this value of 25 may be one element of behavioural vector. Another element can be, for instance, percentile of number of hours that the user's slept, another element can be the number of locations that the user has visited today, etc. In the same manner, humidity level and outdoor temperature can be two elements of ambient vector, also expressed as percentiles depending on the distribution of these parameters at the location where the user lives.

In an embodiment, using the interactive communication module (user's communication module, e.g. user's mobile phone), the system periodically capture semi-static SWB (posing questions to the user as "Overall, how satisfied are you with your life nowadays?", "Overall, to what extent do you feel the things you do in your life are worthwhile?"), and dynamic SWB (with questions such as "How happy did you feel?", "How worthwhile did this feel?" referring to the ongoing activity). The answers to these questions are used as a ground-truth information for the model development and calibration. As previously explained, in the present invention the need to have frequent self-reports in order to monitor SWB and understand its drivers, is reduced (as said question are only posed from time to time in order to develop the model and calibration and not for directly obtaining the user's SWB as in the prior art).

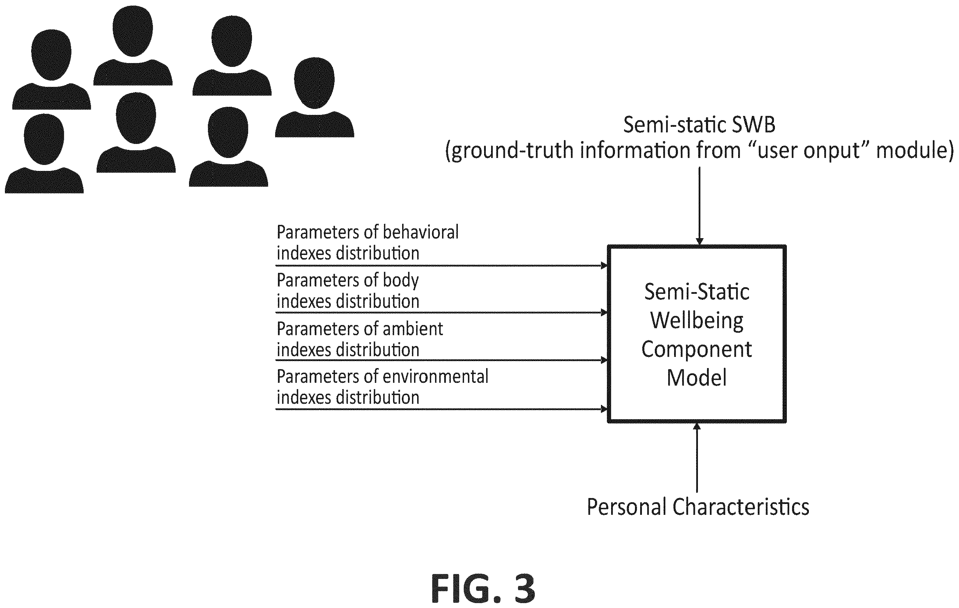

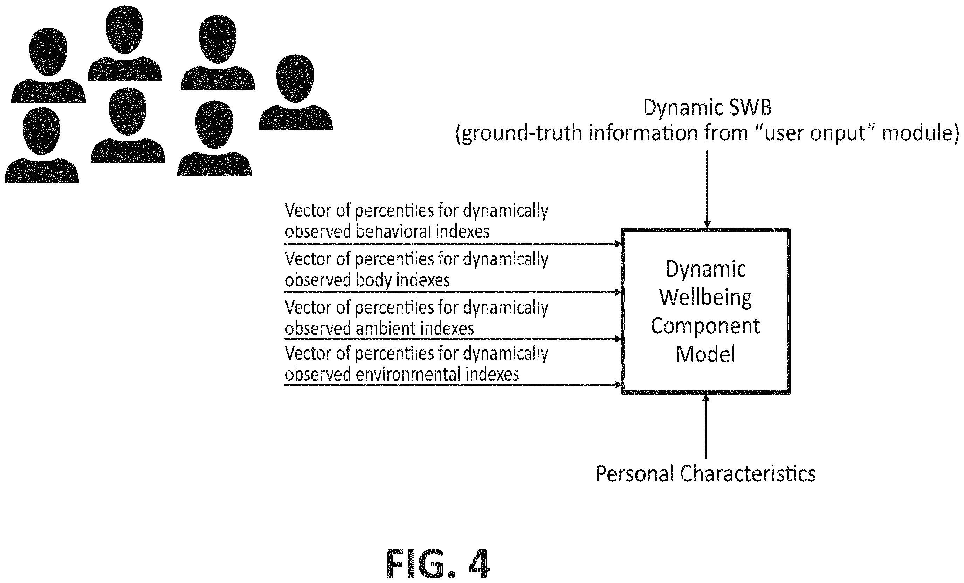

Figures 3 and 4 present the development of semi-static and dynamic SWB models using the data base of users who use the proposed system to estimate their SWB, according to a preferred embodiment of the invention. As shown in figure 3, mapping longitudinal characterization of behavioural, body, ambient and/or environmental parameters (the extracted distribution parameters, figure 1) of all the users in the database (or a significant group of them), optionally together with personal characteristics of all the users in the database (or a significant group of them), to semi-static SWB self-reported by all the users in the database (or a significant group of them) is used by a computer processor to generate a probabilistic model for semi-static SWB (i.e., reflective SWB component, typically referred to as "life-satisfaction" in the literature), called the Semi-Static Wellbeing Component Model. That is, a model to estimate the semi-static SWB component of an user's SWB is built by using the statistic distribution parameters of the monitored (behavioural, body, ambient and/or environmental) data of the users (all the users in the database or a significant group of them), using optionally the personal characteristic of the users and using the semi-static SWB self-reported by the users as ground-data to adjust the model (using computer based techniques). For example, this block may use a machine-learning model (using artificial intelligence) which is trained with the available data.

In an embodiment, the models are created by supervised machine-learning classification algorithms, such as Bayesian Networks, Linear Regression, Support Vector Machines, Decision Trees (e.g. XGBOOST, Random Forests, ...), Hidden Markov Models or any other algorithm. The model can be tuned to balance precision.

Similarly, as shown in figure 4, mapping vectors of percentiles for dynamically observed behavioural, body, ambient and environmental values from the users, optionally together with personal characteristics of the users, to dynamic SWB self-reported by all the users (or a significant group of them) in the data-base generates a probabilistic model for dynamic SWB (i.e., experiential wellbeing) called the Dynamic Wellbeing Component Model. That is, a model to estimate the dynamic SWB component of a user's SWB is built by using the percentiles of the monitored (behavioural, body, ambient and/or environmental) data of the users (all the users in the database or a significant group of them), using optionally the personal characteristic of the users and using the dynamic SWB self-reported by the users as ground-data to adjust the model (using computer based techniques). For example, this block may use a machine-learning model which is trained with the available data.

Hence, the Semi-Static and Dynamic SWB Component Models are generated using information from all the users in the database or a significant group of them, i.e. the model is derived using information not from a specific user but from a group of users. In order to estimate the Semi-Static SWB Component (score) and the Dynamic SWB Component (score) of a specific user, each model is queried with the data of said specific user. Parameters of ambient, environmental, body, behavioural data distributions (and optionally also personal characteristics) of the user will be given as an input to the Semi-Static SWB Component model to obtain the (estimated) Semi-Static SWB score for said specific user, and vectors of percentiles of ambient, environmental, body, behavioural data (and optionally also personal characteristics) of the user will be given as an input to the Dynamicc SWB Component model to obtain the (estimated) Dynamic SWB score for said specific user.

The group of users whose data is used for building the semi-static SWB or the dynamic SWB models can be all the users of the data base or any group of users to which the user may be classified or may have something in common. For example, users using the same service, users to whom the wellbeing is also being estimated, users from the same location (city, neighbourhood...) of the user, users with the same professional activity of the user...or any combination of them. By using data from all the users in the data-base (or a significant group of them), a semi-static and a dynamic model are developed that map all the input variables (or a significant part of them) with SWB. For a new user, when the system only have (ambient, environmental, body, behavioural) input variables (e.g. in the preferred embodiment all passively collected) for said user, the said model can be used to infer semi-static or dynamic SWB of the user without the need to ask him/her for a direct input.

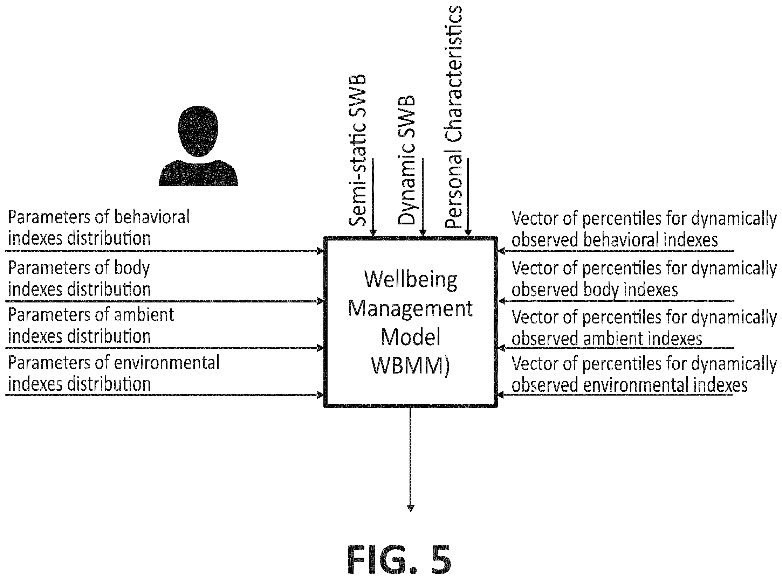

The Wellbeing Management Model (WBMM) is an artificial intelligence driven module (a functional block usually performed by a computer processor) that relies on data obtained or estimated (using other models) for the user (explained in the previous paragraphs) as an input to produce a set of recommendations for improving SWB. As shown in figure 5, the input of this module will be the distribution parameters of user's behavioural, body, ambient and/or environmental data previously extracted, the vector of percentiles extracted for dynamically observed behavioural, body, ambient and/or environmental data previously extracted, the semi-static SWB component (score) estimated for the user (using the previously explained Semi-Static Wellbeing Component Model), the dynamic SWB component (score) estimated for the user (using the previously explained Dynamic Wellbeing Component Model) and optionally the personal characteristics of the user to obtain a recommendation as a range of values for behavioural, body, ambient and environmental parameters to improve the SWB of the user. That is, this module obtains the range of values which the behavioural, body, ambient and environmental parameters of the user must have in order to obtain a better subjective wellbeing.

Through the distribution parameters (left hand side inputs in Figure 5) WBMM learns what typical patterns of behavioural, physiological and surrounding parameters are for an individual in order to capture how variations in specific time periods (percentile measures of dynamically observed indexes) impact SWB that is captured in the previous models. Note that the WBMM (Fig 5) relies on SWB (semi-static and dynamic) model extracted from the whole data-set, however the recommendation (though by using similar inputs to SWB model) is tuned to an individual. Recommendation can be seen as a range of values for behavioural, body, ambient and environmental parameters and their combination that in the model simulation result in an improved SWB. In order to derive personalised recommendations, the method can rely on performing a grid search, i.e., iteratively changing behavioural parameters initiating from small to bigger changes until the positive impact on well-being peaks. For example, this block may use a machine-learning model which is trained with the available data.

Even though, in the embodiments and figures all type of parameters (environmental, sensed behaviours, body signals or ambient parameters) is not mandatory to use parameters from these four types. In a preferred embodiment, all the four type of parameters are used (environmental, sensed behaviours, body signals or ambient parameters). In other embodiments, only one or several of these type of parameters are used (generally speaking the more type and amount of parameters used, the more accurate the estimation of the SWB score will be but also the more resources will be spent)

Depending on the embodiment, these modules (applying the different models) are in the same or in different physical places (different devices). In an embodiment, all modules are performed in the same device (for example, a central or remote server). In other embodiments, the modules (except the mobile communications device itself) are residing on one or more servers. In other embodiments, some modules may be in the user's communications device and the rest in one or more servers. In the case that the modules are in a server, the communications device communicates with the server through a communication networks (a wireless communications network, a mobile communications network as 2G, 3G, 4G or LTE, LAN or W-LAN or any other type of communication networks...).

The description and drawings merely illustrate the principles of the invention.

Although the present invention has been described with reference to specific embodiments, it should be understood by those skilled in the art that the foregoing and various other changes, omissions and additions in the form and detail thereof may be made therein without departing from the scope of the invention as defined by the following claims. Furthermore, all examples recited herein are principally intended expressly to be only for pedagogical purposes to aid the reader in understanding the principles of the invention and the concepts contributed by the inventor(s) to furthering the art, and are to be construed as being without limitation to such specifically recited examples and conditions. Moreover, all statements herein reciting principles, aspects, and embodiments of the invention, as well as specific examples thereof, are intended to encompass equivalents thereof.

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

## Claims

1. A computer implemented method for efficiently estimating and improving the wellbeing state of a user of a group of users, the method comprising:
a) A first electronic device receiving data related to the user acquired by one or more electronic devices, said data being at least one of: ambient data related to a location of the user, environmental data related to the location of the user, physiological data of the user's body and/or behavioural data of the user;
b) The first electronic device obtaining statistic parameters characterising said acquired data related to the user, said statistic parameters being extracted by processing the data related to the user corresponding to a certain period of time;
c) The first electronic device applying at least one probabilistic model to said extracted statistic parameters to obtain an estimated wellbeing score for the user;
d) Determining, using artificial intelligence, a recommended range of values for parameters related to the user which will result on an improvement of the wellbeing score of the user, based at least on the statistic parameters obtained in step b) and on the estimated wellbeing score of the user obtained in step c).

2. A method according to claim 1, where the at least one probabilistic model is generated and adjusted based on periodically received wellbeing information of all the users of the group, directly obtained from each user of the group through an user interface of a communications device of each user; and on statistic parameters extracted by processing data related to all the users of the group, said data being at least one of: ambient data related to a location of the users, environmental data related to the location of the users, physiological data of the users' body and/or behavioural data of the users.

3. A method according to claim 2 where, directly obtaining wellbeing information of all the users of the group comprises: periodically presenting to each user of the group, through the user interface of the communications device of each user, a questionnaire previously received in the communications device of each user; and receiving, through the user interface of the communications device of each user, answers to said questionnaire from each user of the group.

4. A method according to any of the previous claims 2-3 where the communications device of each of the users of the group is one of the following: a laptop, a tablet, a personal computer, a portable computer, a mobile phone or a smart phone.

5. A method according to any previous claim, wherein the statistic parameters are of at least two types: parameters characterising the statistic distribution of the data related to the user during said certain period of time and at least one vector of percentiles values characterising the data related to the user during said certain period of time.

6. A method according to claim 5, where in step c) a semi-static wellbeing score and a dynamic subjective well-being score is estimated and the estimated semi-static wellbeing is obtained applying a first probabilistic model to the parameters characterising the statistic distribution of the data related to the user during said certain period of time and the estimated dynamic wellbeing is obtained applying a second probabilistic model to the at least one vector of percentiles values characterising the data related to the user captured during said certain period of time.

7. A method according to any of the previous claims, where the first electronic device is a remote server and the one or more electronic devices acquiring the data related to the user sent through a communications network the acquired data to the server.

8. A method according to any of the previous claims where data belonging to the fourth types of data: ambient data related to a location of the user, environmental data related to the location of the user, physiological data of the user's body and behavioural data of the user, are acquired and received by the first electronic device in step a) and used in step b) and d).

9. A method according to any of the previous claims where the recommended range of values for parameters related to the user is sent by the first electronic device to the user's communication device through a communications network.

10. A method according to any of the previous claims where personal characteristics of the user are also taken into account in step c) by the one or more probabilistic models to obtain the estimated wellbeing score of the user and/or are also taken into account in step d) to determine the recommended range of values for parameters related to the user.

11. A method according to any of the previous claims where the environmental data are values of at least one of the following parameters: pollution, outdoor light level, light level, weather, humidity, outdoor temperature; the behavioural data are values of at least one of the following parameters; mobility, social interactions, sleep state; the physiological data are values of at least one of the following parameters of the user's body: galvanic skin response, heart rate variability, skin temperature; and the ambient data are values of at least one of the following parameters: indoor temperature, indoor light level, light exposure, noise level.

12. A method according to any of the previous claims where one or more electronic devices acquiring data signals related to the user are at least one of the following: usage logs or sensors embedded in a communications device of the user, wearable electronic devices with sensors to measure body signals, presence sensors, location sensors, smart environments devices, bed-sensors, environment sensors to monitor environment parameters such as temperature, air quality or weather information.

13. An electronic system for efficiently estimating and improving the wellbeing state of a user of a group of users, the system comprising:
- Communication means for receiving, through a communications network, data related to the user acquired by one or more electronic devices, said data being at least one of: ambient data related to a location of the user, environmental data related to the location of the user, physiological data of the user's body and/or behavioural data of the user;
- Processing means configured to:
- Obtain statistic parameters characterising said acquired data related to the user, the statistic parameters being extracted by processing the data related to the user corresponding to a certain period of time;
- Apply at least one probabilistic model to said extracted statistic parameters to obtain an estimated wellbeing score for the user;
- Based at least on the extracted statistic parameters characterising the data related to the user and the estimated wellbeing score of the user, determine using artificial intelligence a recommended range of values for parameters related to the user which will result on an improvement of the wellbeing score of the user.

14. A system according to claim 13, wherein the communication means are further configured to receive through the communications network wellbeing information of all the users of the group, directly obtained from each user of the group through an user interface of a communications device of each user; wherein the communication means are further configured to receive through the communications network data related to all the users of the group, said data being at least one of: ambient data related to a location of the users, environmental data related to the location of the users, physiological data of the users' body and/or behavioural data of the users; and where the at least one probabilistic model is generated and adjusted by the processing means based on the received wellbeing information of all the users of the group and on the received data related to all the users of the group.

15. A non-transitory computer readable medium, comprising instructions for causing a computer device to perform the method of any of the claims 1-12.
